# EUROPÄISCHE PATENTANMELDUNG

(11) **EP 1 089 228 A1**
(43) Veröffentlichungstag der Anmeldung: **04.04.2001**
(21) Anmeldenummer: 00114952.5
(22) Anmeldetag: 19.07.2000
(51) Int. Cl.: G06T 7/00, A61B 6/00, A61B 5/055

(54) **Kontinuierliche Erfassung und Analyse von Gewebeveränderungen**

(30) Priorität: 28.09.1999 DE 19946429
(71) Anmelder: Vilsmeier, Stefan, 6330 Kufstein (AT)
(72) Erfinder: Vilsmeier, Stefan, 6330 Kufstein (AT)
(74) Vertreter: Schwabe - Sandmair - Marx

(57) **Zusammenfassung**

Die Erfindung betrifft ein Verfahren zur Erfassung von Gewebeveränderungen mit dem Schritten: a) eine Gewebestrukturerfassung wird für mindestens einen Teil eines Patientenkörpers erstellt und alle Daten aus der Gewebestrukturerfassung werden gespeichert; b) nach einer vorbestimmten Zeitspanne wird mindestens einmal für den Teil bzw. den gesamten Patientenkörper nochmals eine Gewebestrukturerfassung erstellt, deren Daten wiederum gespeichert werden; c) die Daten zweier oder mehrerer, nacheinander erfolgter Gewebestrukturerfassungen werden computergestützt positionell zugeordnet und verglichen; d) Veränderungen des Patientengewebes, die sich aus unterschiedlichen Daten für jeweilige zugeordnete Körperabschnitte ergeben, werden computergestützt erfasst und ausgegeben.

## Beschreibung

Die vorliegende Erfindung betrifft ein Verfahren zur Erfassung von Gewebeveränderungen.

Die Früherkennung von Gewebeveränderungen ist in der Medizin und insbesondere bei der Krebsbekämpfung sehr wichtig, da sich bei einer möglichst frühen Feststellung von Gewebeveränderungen meistens noch die Möglichkeit ergibt, Maßnahmen zu ergreifen, die zur völligen Gesundung eines Patienten führen. Bekannte Früherkennungsmethoden sind die Entnahme und Analyse von Körperflüssigkeiten, die Abtastung oder in der Apparatemedizin auch die Betrachtung von Tomographieaufnahmen sowie Ultraschallaufnahmen einzelner Körperteile.

All diese Methoden haben zunächst den Nachteil, dass sie nur "Momentaufnahmen" liefern. Der Arzt kann hierbei zwar den momentanen Zustand des Gewebes erkennen, jedoch hat er nicht die Möglichkeit ein Wachstum einer Gewebeveränderung festzustellen. Oft ist es auch für geübte Ärzte schwierig, aus einem solchen einzelnen Bild krankhafte Gewebsveränderungen auszulokalisieren.

Es ist deshalb die Aufgabe der vorliegenden Erfindung, ein Verfahren zur Erfassung von Gewebeveränderungen bereitzustellen, welches die oben aufgeführten Nachteile überwindet. Insbesondere sollen Form- und Größenänderungen solcher Gewebeveränderungen leicht erfassbar gemacht werden. Dem behandelnden Arzt soll ein technisches Mittel zur Verfügung gestellt werden, mittels welchem er in relativ einfacher Weise den Fortschritt bzw. das Entstehen von Gewebeveränderungen analysieren kann.

Die Erfindung stellt dazu ein Verfahren zur Erfassung von Gewebeveränderungen mit den folgenden Schritten zur Verfügung:
a) eine Gewebestrukturerfassung wird für mindestens einen Teil eines Patientenkörpers erstellt und alle Daten aus der Gewebestrukturerfassung werden gespeichert;
b) nach einer vorbestimmten Zeitspanne wird mindestens einmal für den Teil bzw. den gesamten Patientenkörper nochmals eine Gewebestrukturerfassung erstellt, deren Daten wiederum gespeichert werden;
c) die Daten zweier oder mehrerer, nacheinander erfolgter Gewebestrukturerfassungen werden computergestützt positionell zugeordnet und verglichen;
d) Veränderungen des Patientengewebes, die sich aus unterschiedlichen Daten für jeweilige zugeordnete Körperabschnitte ergeben, werden computergestützt erfasst und ausgegeben.

Mit anderen Worten wird also in bestimmten Zeitabständen über eine längere Zeitdauer immer wieder eine Gewebestrukturerfassung eines Teils eines Patientenkörpers oder eines gesamten Patientenkörpers erstellt. Nachdem der Computer die übereinstimmenden Körperteile zugeordnet und verglichen hat, können dann Veränderungen im Gewebe festgestellt und so ausgegeben werden, dass sie einfach erkennbar sind. Dadurch wird es möglich, einen sich bildenden oder wachsenden Krankheitsherd im Körper früh aufzufinden und dessen Entwicklung zu beobachten.

Wenn gemäß einer Ausführungsform der Erfindung der gesamte Körper erfasst wird, besteht nicht mehr die Gefahr, dass ein relativ junger Krankheitsherd übersehen wird und es können frühzeitig therapeutische Maßnahmen eingeleitet werden. Die Zuordnung der einzelnen Körperpunkte und der Vergleich zweier nacheinander erfolgter Erfassungen wird vom Computer übernommen, und weil die Gewebeveränderungen in einfach sichtbarer Weise ausgegeben werden können, lässt sich eine eventuell krankhafte Läsion auch von weniger geübten Ärzten erkennen. Damit wird eine umfassende Früherkennung möglich und Heilungschancen, insbesondere bei Krebskrankheiten, werden drastisch erhöht.

Die Gewebestrukturerfassung kann eine Tomographie, einerseits eine Computertomographie, andererseits auch eine Kernspintomographie sein. Ferner kann die Gewebestrukturerfassung auch eine Erfassung aus dem Bereich der Nuklearmedizin sein, eine Knochenscintigraphie, eine Mammographieaufnahme, eine Ultraschallabtastung, eine PET- oder eine SPECT- Erfassung. Im Grund ist die Verwendung aller Erfassungen und medizinischer Diagnosescans denkbar, die ein Abbild einer Gewebestruktur liefern können. Natürlich können auch Daten aus allen oben genannten Erfassungsverfahren kombiniert werden, um eine möglichst vollständige Patientenerfassung zu gewährleisten.

Der Einsatz einer Kernspintomographie ist von besonderem Vorteil. Da das erfindungsgemäße Verfahren darauf aufbaut, in gewissen Zeitabständen immer wieder eine vollständige oder teilweise Erfassung des Patientenkörpers vorzunehmen, muss darauf geachtet werden, dass die Strahlenbelastung möglichst gering gehalten wird. Die Kernspintomographie gestattet eine solche Abtastung mit geringer Belastung und ist in letzter Zeit auch mit weniger Aufwand realisierbar geworden.

Vorzugsweise erfolgt der Vergleich der zugeordneten Gewebedaten erfindungsgemäß durch eine Subtraktion der Bilddaten aus verschiedenen Erfassungen. Hierzu kann ein Bildverarbeitungsprogramm verwendet werden.

Gemäß einer besonders bevorzugten Ausführungsform des erfindungsgemäßen Verfahrens werden nach dem Vergleich die Gewebeveränderungen sichtbar und unterscheidbar auf einem Bildschirm ausgegeben. Dabei ist es beispielsweise von Vorteil, wenn die auffälligen Gewebeveränderungen farblich abgehoben dargestellt werden. Die nicht veränderten Körperteile können ferner transparent bzw. halbtransparent so ausgegeben werden, dass die Veränderungen und ihre Positionen sichtbar sind.

Vorzugsweise wird hierzu eine dreidimensionale Animation des Körpers verwendet. Bei einer solchen Ausgabe entsteht ein "gläserner Mensch", wobei im Körperinneren lediglich die veränderten Gewebestellen sichtbar gemacht werden, beispielsweise farbig hervorgehoben werden. Auch ein relativ ungeübter Mediziner kann in einem solchen Bild krankhafte Gewebsläsionen erkennen und deren Fortschritt beobachten. Natürlich besteht grundsätzlich die Möglichkeit, zu den hervorgehobenen Gewebeveränderungen noch halbtransparent feste Punkte im Körper auszugeben, beispielsweise das Knochenskelett, um die Lokalisation zu vereinfachen.

Vorteilhafterweise können die Positionsdaten der Gewebeveränderungen, welche durch die Erfassung(en) vorliegen, bei anschließenden Behandlungen zur Lokalisation verwendet werden.

Die positionelle Zuordnung der Daten aus verschiedenen Gewebestrukturerfassungen erfolgt gemäß einer erfindungsgemäßen Ausführungsform dadurch, dass das Volumen des Körperabschnittes aus einer Erfassung als ein Stück elastisches Material modelliert wird, wobei auf die Form des elastischen Materialstückes virtuelle äußere Kräfte zur Deformation aufgebracht werden, bis eine Übereinstimmung zwischen den zuzuordnenden Körperabschnitten aus den Erfassungen erzielt ist. Die Datensätze aus den Erfassungen können dabei zunächst vorab global ausgerichtet werden.

Ferner sind vorzugsweise die virtuellen äußeren Kräfte zur Deformation so lange aufzubringen, bis ein Gleichgewicht zwischen den äußeren Kräften und inneren Reaktionskräften entsteht, wobei insbesondere die Deformation in einer Grob-zu-Fein-Strategie solange fortschreitet, bis die äußeren Kräfte ein Minimum annehmen.

Eine Ausführungsform des erfindungsgemäßen Verfahrens hat im wesentlichen den folgenden Ablauf:

Der Patient begibt sich zu einem Arzt bzw. zu einer anderen Einrichtung, bei der ein Kernspintomograph zur Verfügung steht. Nunmehr wird bei der ersten Erfassung der gesamte Körper des Patienten oder ein Teil davon mittels der Kernspintomographie gescannt und die Daten werden abgespeichert. Eventuell können schon bei dieser ersten Erfassung krankhafte Gewebsläsionen erkannt, und zur weiteren Beobachtung bzw. schon zu einer ersten Behandlung können diese Daten verwendet werden.

Nach einer bestimmten Zeitdauer, die im Falle eines gesunden Körpers etwa ein halbes Jahr betragen kann und welche bei Verdacht auf krankhafte Veränderungen entsprechend abzukürzen ist, begibt sich der Patient wiederum zur Tomographie und ein zweiter Scan wird erstellt.

Hierauf erfolgt der computergestützte Datenabgleich und die Erfassung und Ausgabe der Gewebeveränderungen. Dies kann dadurch geschehen, dass die Patientendaten an eine zentrale Stelle weitergegeben werden, welche die entsprechende Computerausrüstung aufweist oder, bei entsprechender technischer Ausstattung der Einrichtung vor Ort, gleich nach der Tomographieerfassung.

Die in den beiden Tomographieerfassungen erhaltenen Daten über den Patientenkörper werden nunmehr computerunterstützt zunächst positionell zugeordnet. Dies bedeutet, dass die beiden Abbilder zunächst "übereinandergelegt" werden, so dass die jeweiligen Körperpunkte im ersten Datensatz wieder den entsprechenden Körperpunkten im zweiten Datensatz zugeordnet werden. Eine solche Zuordnung kann z.B. wie folgt durchgeführt werden:

Ein früherer Bildsatz eines Patienten wird einem Abgleich mit einem aktuellen Bildsatz unterzogen (Morphing). Die Annahme, die hierbei getroffen wird, ist, dass alle menschlichen Körperteile zumindest bis zu einem bestimmten Darstellungsgrad dieselbe topologische Struktur aufweisen, aber im Formdetail verändert sein können, wie dies beispielsweise bei einem wachsenden Tumor der Fall ist.

Zuerst werden zwei Bildsätze global unter Verwendung eines Algorithmus für eine starre Bildregistrierung ausgerichtet. Nach dieser globalen Ausrichtung wird das Volumen, das von einem der Bildsätze eingenommen wird, wie ein Stück elastisches Material modeliert. Durch das Aufbringen von äußeren Kräften kann die Form des elastischen Objektes verändert werden, bis es sich dem Referenzobjekt angepaßt hat. Als Resultat dieser Kräfte wird das Objekt deformiert, bis ein Gleichgewichtszustand zwischen den äußeren Kräften und den inneren elastischen Rückstellkräften erzielt ist. Die Deformation schreitet in einer Grobzu-Fein Strategie fort, wobei die lokale Angleichung und die globale Kohärenz gesteigert werden.

Die Ableitung der äußeren Kräfte ist der wichtigste Teil des elastischen Anpassungsmodels. Die Aufgabe dieser Kräfte ist es, gleiche Regionen in beiden Bildsätzen zur Übereinstimmung zu bringen. Der gegenseitige Informationsaustausch ist hier das geeignete Mittel zur Messung des lokalen Angleichungsgrades.

Das Problem der elastischen Übereinstimmungsherstellung kann als Optimierungsproblem der Kostenfunktion 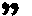 Kosten = Deformation - Angleichungsgrad" formuliert werden. Es ist dabei die Aufgabe, äußere Kräfte zu finden, bei denen die Kostenfunktion ein Minimum einnimmt. Die Verschiebung jedes Punktes des Objektes wird durch einen gekoppelten Satz partieller Differenzialgleichung geregelt, welche die äußeren Kräfte und einige Parameter umfassen, die das elastische Verhalten des Models beschreiben. Diese partiellen Differenzialgleichungen können mit Hilfe einer Finite-Elemente-Methode gelöst werden, wobei die bereits existierende räumliche Diskretisierung in Volumenelemente (Voxels) in Betracht gezogen wird. Die genannte 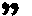 Grob-zu-Fein-Strategie" vermindert die Rechenzeit und reduziert das Risiko der Konvergenz an einem lokalen Minimum.

Nachdem die beiden Datensätze aus den Tomographieerfassungen nun zugeordnet sind, wird ebenfalls computerunterstützt ein Vergleich der einzelnen Abschnitte durchgeführt, wobei diejenigen Daten, bei denen keine Veränderung vorliegt, subtrahiert werden, so dass nur noch die Veränderungen und ihre Positionen sichtbar sind. Durch entsprechende Filter können hierbei Veränderungen vernachlässigt werden, die mit Sicherheit nicht krankhaft sind, so dass am Ende dieses Abgleichs ein Bild, insbesondere ein animiertes Ganzkörperbild des Patienten ausgegeben werden kann, in dem nur noch verdächtigte Gewebeveränderungen ersichtlich sind. Ein großer Vorteil hierbei besteht darin, dass auch das Wachstum solcher Gewebeveränderungen erkenntlich wird. Auch langsam wachsende, sehr junge Krebsgeschwüre können Somit erkannt und frühzeitig behandelt werden, was die Heilungschancen stark vergrößert.

Zur Vorbeugung bzw. zur weiteren Verfolgung des Fortschrittes behandelter und nicht behandelter Gewebeveränderungen wird dann das oben erläuterte Vorgehen in vorbestimmten Zeitabständen wiederholt.

## Patentansprüche

1. Verfahren zur Erfassung von Gewebeveränderungen mit den folgenden Schritten:
a) eine Gewebestrukturerfassung wird für mindestens einen Teil eines Patientenkörpers erstellt und alle Daten aus der Gewebestrukturerfassung werden gespeichert;
b) nach einer vorbestimmten Zeitspanne wird mindestens einmal für den Teil bzw. den gesamten Patientenkörper nochmals eine Gewebestrukturerfassung erstellt, deren Daten wiederum gespeichert werden;
c) die Daten zweier oder mehrerer, nacheinander erfolgter Gewebestrukturerfassungen werden computergestützt positionell zugeordnet und verglichen;
d) Veränderungen des Patientengewebes, die sich aus unterschiedlichen Daten für jeweilige zugeordnete Körperabschnitte ergeben, werden computergestützt erfasst und ausgegeben.

2. Verfahren nach Anspruch 1, bei dem die Gewebestrukturerfassung eine Tomographie, insbesondere eine Computertomographie oder eine Kernspintomographie ist.

3. Verfahren nach Anspruch 1, bei dem die Gewebestrukturerfassung eine Erfassung aus dem Bereich der Nuklearmedizin ist, eine Knochenscintigraphie, eine Mammographieaufnahme, eine Ultraschallabtastung, eine PET- oder eine SPECT- Erfassung ist.

4. Verfahren nach Anspruch 1, bei dem Daten aus den in den Ansprüche 2 und 3 genannten Erfassungen kombiniert verwendet werden.

5. Verfahren nach einem der Ansprüche 1 bis 4, bei dem der Vergleich durch eine Subtraktion der Bilddaten aus verschiedenen Erfassungen erfolgt.

6. Verfahren nach einem der Ansprüche 1 bis 5, bei dem nach dem Vergleich auffällige Gewebeveränderungen sichtbar und unterscheidbar auf einem Bildschirm ausgegeben werden.

7. Verfahren nach Anspruch 6, bei dem die auffälligen Gewebeveränderungen farblich abgehoben dargestellt werden.

8. Verfahren nach Anspruch 6 oder 7, bei dem die nicht veränderten Körperabschnitte transparent bzw. halbtransparent so ausgegeben werden, dass die Veränderungen und ihre Positionen sichtbar sind.

9. Verfahren nach einem der Ansprüche 1 bis 6, bei dem die Ausgabe als dreidimensionale Animation des Körpers bzw. Körperteils erfolgt.

10. Verfahren nach einem der Ansprüche 1 bis 7, bei dem die Positionsdaten der Gewebeveränderungen bei anschließender Behandlung zur Lokalisation verwendet werden.

11. Verfahren nach einem der Ansprüche 1 bis 10, bei dem die positionelle Zuordnung der Daten aus verschiedenen Gewebestrukturerfassungen dadurch erfolgt, dass das Volumen des Körperabschnittes aus einer Erfassung als ein Stück elastisches Material modelliert wird, wobei auf die Form des elastischen Materialstückes virtuelle äußere Kräfte zur Deformation aufgebracht werden, bis eine Übereinstimmung zwischen den zuzuordnenden Körperabschnitten aus den Erfassungen erzielt ist.

12. Verfahren nach Anspruch 11, bei dem die Datensätze aus den Erfassungen zunächst vorab global ausgerichtet werden.

13. Verfahren nach Anspruch 11 oder 12, bei dem die virtuellen äußeren Kräfte zur Deformation so lange aufgebracht werden, bis ein Gleichgewicht zwischen den äußeren Kräften und inneren Reaktionskräften entsteht.

14. Verfahren nach einem der Ansprüche 11 bis 13, bei dem die Deformation in einer Grob-zu-Fein-Strategie fortschreitet, bis die äußeren Kräfte ein Minimum annehmen.
